# EUROPEAN PATENT SPECIFICATION

(11) **EP 4 490 144 B1**
(45) Date of publication and mention of the grant of the patent: **18.11.2026**
(21) Application number: 23713755.9
(22) Date of filing: 07.03.2023
(51) Int. Cl.: C07C 403/24

(54) **METHOD OF EXTRACTION AND RECOVERY OF ASTAXANTHIN FROM BIOMASS**
VERFAHREN ZUR EXTRAKTION UND RÜCKGEWINNUNG VON ASTAXANTHIN AUS BIOMASSE
PROCÉDÉ D'EXTRACTION ET DE RÉCUPÉRATION D'ASTAXANTHINE À PARTIR D'UNE BIOMASSE

(30) Priority: 07.03.2022 IT 202200004247
(43) Date of publication of application: 15.01.2025
(73) Proprietor: Universita'Degli Studi di Roma "La Sapienza", 00185 Roma (IT)
(72) Inventor: BRAVI, Marco, 00136 Roma (IT); SCARSELLA, Marco, 00175 Roma (IT); SAPONE, Vittoria, 00175 Roma (IT)
(74) Representative: Cattaneo, Elisabetta
(86) International application number: PCT/IB2023/052124
(87) International publication number: WO 2023/170564

(56) References cited:
- CN-A- 104 557 649
- CN-A- 105 732 452
- US-A- 5 356 810

## Description

### FIELD OF THE INVENTION

The present invention concerns a method of extraction and recovery of astaxanthin from biomass.

### STATE OF THE ART

Antioxidants may be defined as molecules which, at low concentrations, delay or prevent oxidation, by acting at the level of biological membranes, or at the intracellular level, thus protecting cells of different organs and different biological systems. Among natural antioxidants, carotenoids and their derivatives stand out as a large group of molecules naturally produced by plants and other photosynthetic organisms. These molecules are capable of protecting cells from light-mediated oxidative processes, and peroxidation mediated by free radicals or singlet oxygen. Among them, astaxanthin is a compound of formula I: 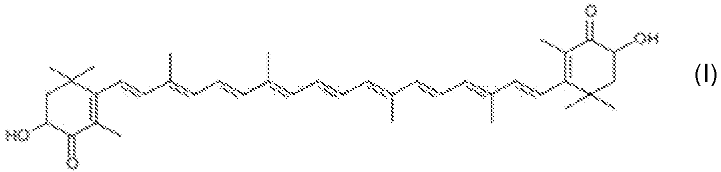 and it is one of the carotenoids produced by aquatic organisms most widespread in nature, which stands out in its chemical family for its high oxygen radicals absorption capacity, with an antioxidant capacity 100-500 times greater than α-tocopherol (Vitamin E), a well-known antioxidant. Several natural sources of astaxanthin have been reported, including the microalgae *Haematococcus pluvialis, Chlorella vulgaris, Chlorella zoofingiensis* and *Chlorococcum sp.,* the red yeast *Phaffia rhodozyma,* as well as the bacterium *Paracoccus carotinifaciens sp. nov.,* just to name a few. Superior organisms (fish and shellfish, first of all) that feed on these microalgae accumulate the carotenoid in their tissues and take the typical red or pink color. Consequently, astaxanthin is also contained in waste from the shellfish processing industry (which represents about 40-50% by weight of the raw material processed).

In addition to that from biomass, synthetic astaxanthin from raw materials of fossil origin is commercially available. To date, it is estimated that approximately 80% of commercially available astaxanthin comes from fossil sources.

However, biomass and synthetic astaxanthin are not two completely identical products in terms of chemical composition, bioavailability, purity, or organoleptic qualities. Biomass astaxanthin exists in fact in free and esterified form, and as a mixture of its three stereoisomers (3S,3'S; 3R,3'R; 3R,3'S) present in variable amounts specific to the organism producing it, while the synthetic one is a racemic mixture of three isomers (3S,3'S⁻ 3R,3'S: 3R,3'R = 1:2:1) and may usually contain traces of residual solvents and chemical reagents.

Biomass astaxanthin also exhibits superior biological activities and was initially recognized as a fish feed additive (to improve both the coloration of ornamental aquarium fish and the meat color of farmed salmon and trout for human consumption), gaining market as an ingredient for supplements and cosmetic products.

Astaxanthin produced by *Haematococcus pluvialis,* for example, is commonly used for human application (Kitamura 2015; Shah et al. 2016), the one produced by *Phaffia rhodozyma* is mainly used for fish pigmentation, meat and eggs, while astaxanthin produced by *Paracoccus carotinifaciens,* originally used for animal feed, has later found application also in the sector of nutraceutical products.

Several studies have then shown a wide range of potential mechanisms through which astaxanthin could exert its beneficial effects, including photoprotective, antioxidant, anti-inflammatory and anti-apoptosis effects, acting at different levels, including beneficial effects for the skin, the cardiovascular system and the eyes. Furthermore, several studies have demonstrated its neuroprotective capacity and antitumor activity.

Astaxanthin is therefore a carotenoid of ever-increasing interest in various industrial sectors and the need to improve the technologies for its production is particularly felt, in particular the obtaining thereof from natural sources such as biomass.

To date, solvent extraction is a widely implemented method to obtain high-value metabolites, such as astaxanthin, from biomass. Currently, methods for extracting astaxanthin are known and used, such as acetone, ethyl acetate, chloroform/methanol, chloroform and hexane. Sarada (Sarada et al., J Agr Food Chem 54.20, 2006, 7585-7588.) reports the results of the extraction of astaxanthin from *H. pluvialis* using various solvents (acids and DMSO), methanol and acetone (hydrochloric acid, acid citric acid, tartaric acid, acetic acid, and formic acid). A treatment with 1-2 N HCl for 5-10 minutes while heating at 70°C, combined with extraction with acetone, allows a recovery of up to 96% of the astaxanthin contained in *H. pluvialis.* The use of the solvent component alone leads to a recovery of about 19% of astaxanthin. Effective recovery of astaxanthin from cells characterized by a resistant wall, such as those from *H. pluvialis,* indeed requires means which, alone or in combination, are capable of breaking the cell wall and acting as a good solvent for intracellular astaxanthin. Dong (Dong et al., Sci World J, 2014) investigated different means to extract astaxanthin from *H. pluvialis,* showing that treatment with HCl followed by acetone extraction performs much better than the other methods evaluated. In another study, Zou (Zou et al., Mar drugs 11.5, 2013, 1644-1655) enhanced the extraction of astaxanthin from dried *H. pluvialis* using a mixture consisting of ethanol and ethyl acetate, in series with an ultrasound treatment aimed at the cell wall destruction step. Zou also noted that extended exposure of cells to organic solvents, necessary to overcome an unfavorable mass transfer coefficient due to cell wall thickness, can significantly deteriorate the stability and quality of astaxanthin. Furthermore, the extraction with volatile solvent is energy intensive and involves high operating costs, in addition to exposure to potential releases when carried out with classical organic solvents (Mercer and Armenta,. Eur J Lip Sci Technol 113.5, 2011, 539-547).

An approach, which is alternative to that with traditional solvents, consists of processes that use vegetable oils, such as soy, corn, olive and grape seed oils (Kang and Sang, Biotechnol Lett 30.3, 2008, 441-444). Despite being a valid alternative for food-grade astaxanthin, this type of extraction process requires a high residence time (>48 hours) and does not allow for the separation of astaxanthin from the oil. In this way, if on the one hand the oil can be exploited as an extraction system, on the other it is bound to a ratio between oil and solute that cannot be further increased.

Another type of solvent, alternative to traditional organic solvents, that has been tested is supercritical CO₂, which from the point of view of the solvent power behaves similarly to them and has some further advantages. While to obtain astaxanthin from a traditional organic solvent this requires to be subsequently evaporated, in the case of supercritical CO₂ the separation is obtained by reducing the pressure below the critical point (73 bar). However, due to the low solubility of astaxanthin in CO₂, very high extraction pressures are required (over 50 MPa) and in fact the supercritical CO₂ extraction methods also provide for the addition of ethanol as a co-solvent, which allows better yields at slightly lower pressures (Wang et al., Inn Food Sci Emerg Technol 13, 2012, 120-127).

Finally, other types of solvents have also been tested, such as ionic liquids (Bi et al., 2010), switchable-hydrophilicity solvents (Huang et al., 2018), or eutectic mixtures such as NaDES, Natural Deep Eutectic Solvent (Sapone, Chem Eng Trans 87, 2021, 463-468).

Although there are various technologies for obtaining astaxanthin from biomass, the Applicant has therefore observed that these currently have a whole series of technical and performance limitations, which make their application not entirely satisfactory. US5356810A discloses in Example 9 the extraction of astaxanthin from the yeast Phaffia rhodozyma with acetic acid.

In particular, the Applicant has observed that processes using traditional organic solvents are particularly affected by a low mass transfer coefficient through the cell wall, thus requiring the adoption of mechanical or chemical pre-treatments or the need to subject the substrate to prolonged exposure of the cells to solvents which can, however, significantly deteriorate the stability and quality of astaxanthin. Furthermore, these are energy-intensive processes and involve high operating costs, as well as the exposure of operators and the environment to potential solvent releases.

The Applicant has also observed that the astaxanthin extraction processes which use vegetable oils as solvents have long extraction times (>48 hours), thus resulting to be poorly competitive, and furthermore that a step of separating the astaxanthin from the oil cannot be implemented in a simple way; this implies a maximum limit on the astaxanthin content in the oil produced and inapplicability on an industrial scale of this method of obtaining astaxanthin in isolated form.

The Applicant has also observed that technologies based on the use of supercritical CO₂ must resort to very high extraction pressures or the addition of co-solvents, thus only partially overcoming the limits of other technologies, while the ones based on other solvents (e.g. ionic liquids, switchable hydrophilicity solvents, NaDES), have limits due to the cost and recovery of the solvents used and also do not allow astaxanthin to be easily separated from the latter, thus also being inapplicable on an industrial scale to obtain astaxanthin in isolated solid form.

The Applicant has therefore found that, to date, there is a need for a technology of obtaining astaxanthin from biomass which overcomes the limits of the technologies known to date.

### SUMMARY OF THE INVENTION

The object of the present invention is therefore to address this need and, in particular, to provide a new method of obtaining astaxanthin from natural sources, such as biomass, which allows its extraction and as well as recovery, does not compromise the stability and quality of the astaxanthin obtained, is competitive in terms of operating times and costs, and makes the recovery of astaxanthin in solid form simple and applicable at low cost.

According to the present invention, the Applicant has surprisingly found that it is possible to pursue this and other advantageous purposes by subjecting an astaxanthin-containing biomass to solid-liquid extraction with a solvent comprising at least one linear carboxylic acid selected from the group consisting of acetic acid, propanoic (or propionic) acid, butanoic (or butyric) acid, pentanoic (or valeric) acid, and hexanoic acid, and subsequently recovering astaxanthin in solid form thanks to the use of a basic aqueous solution capable of leading to its precipitation and easy separation from said solvent.

In particular, in its first aspect, the present invention relates to a method of obtaining astaxanthin from a biomass, comprising the steps of:
a. extracting an astaxanthin-containing biomass with a solvent comprising at least one linear carboxylic acid selected from the group consisting of acetic acid, propanoic acid, butanoic acid, pentanoic acid, and hexanoic acid, thus obtaining a first dispersion comprising an exhausted biomass and a solution comprising astaxanthin;
b. separating in the first dispersion of step a. said solution comprising astaxanthin from said exhausted biomass;
c. contacting the solution comprising astaxanthin from step b. with a basic aqueous solution, thus obtaining a second dispersion comprising a solid comprising astaxanthin and an exhausted aqueous solution; and
d. separating in the second dispersion of step c. said solid comprising astaxanthin from said exhausted aqueous solution, thus obtaining astaxanthin in solid form.

In fact, the Applicant has surprisingly found that the use of a linear carboxylic acid selected from the group consisting of acetic acid, propanoic acid, butanoic acid, pentanoic acid, and hexanoic acid as a solvent, allows to efficiently extract astaxanthin from a biomass and bring it into solution, and furthermore the subsequent addition of a basic aqueous solution leads to the precipitation and easy recovery of astaxanthin in solid form, with excellent of stability and compositional quality characteristics thanks to its biomass origin.

In a further aspect thereof, the present disclosure also relates to astaxanthin in solid form obtainable by the method according to the first aspect of the invention.

In a still further aspect thereof, the present disclosure also relates to the use of astaxanthin in the form obtainable by the method according to the first aspect of the invention, for the preparation of a pharmaceutical product, a nutraceutical product, a cosmetic product, a product for human nutrition, or a product for animal feed.

The advantages and characteristics of these further aspects have already been highlighted with reference to the first aspect of the invention and are not repeated here.

### DETAILED DESCRIPTION OF THE INVENTION

In its first aspect, the present invention relates to a method of obtaining astaxanthin from a biomass, comprising the steps of:
a. extracting an astaxanthin-containing biomass with a solvent comprising at least one linear carboxylic acid selected from the group consisting of acetic acid, propanoic acid, butanoic acid, pentanoic acid, and hexanoic acid, thus obtaining a first dispersion comprising an exhausted biomass and a solution comprising astaxanthin;
b. separating in the first dispersion of step a. said solution comprising astaxanthin from said exhausted biomass;
c. contacting the solution comprising astaxanthin from step b. with a basic aqueous solution, thus obtaining a second dispersion comprising a solid comprising astaxanthin and an exhausted aqueous solution; and
d. separating in the second dispersion of step c. said solid comprising astaxanthin from said exhausted aqueous solution, thus obtaining astaxanthin in solid form.

The Applicant has in fact surprisingly found that it is possible to provide a new method for the extraction and recovery of astaxanthin from natural sources, such as biomass, which does not compromise the stability and quality of the astaxanthin obtained, is competitive in terms of operating times and costs, and makes the recovery of astaxanthin in solid form simple and cost-effective by subjecting an astaxanthin-containing biomass to solid-liquid extraction with a solvent comprising at least one linear carboxylic acid selected from the group consisting of acetic acid, propanoic acid , butanoic acid, pentanoic acid, and hexanoic acid, and subsequently recovering astaxanthin in solid form thanks to the use of a basic aqueous solution capable of leading to its precipitation and easy separation from said solvent.

The Applicant has in fact surprisingly found that the use of a linear carboxylic acid selected from the group consisting of acetic acid, propanoic acid, butanoic acid, pentanoic acid, and hexanoic acid as a solvent, allows to efficiently extract astaxanthin from a biomass and bring it into solution, and furthermore the subsequent addition of a basic aqueous solution leads to precipitation and easy recovery of astaxanthin in solid form, with excellent stability and compositional quality characteristics thanks to its biomass origin.

Within the scope of the present description and in the subsequent claims, all the numerical quantities indicating amounts, parameters, percentages, and so on, are to be understood as preceded in all circumstances by the term "about" unless otherwise indicated.

Furthermore, all numerical quantities ranges include all possible combinations of maximum and minimum numerical values and all possible intermediate ranges, in addition to those specifically indicated below.

The present invention may have in one or more of its aspects one or more of the preferred characteristics reported below, which can be combined with each other according to the application requirements.

The method according to the present invention comprises the step a. of extracting an astaxanthin-containing biomass with a solvent comprising at least one linear carboxylic acid selected from the group consisting of acetic acid, propanoic acid, butanoic acid, pentanoic acid, and hexanoic acid, thus obtaining a first dispersion comprising an exhausted biomass and a solution comprising astaxanthin.

Astaxanthin-containing biomass are known, such as for example the algae *Haematococcus pluvialis, Chlorella vulgaris, Chlorella zoofingiensis* and *Chlorococcum sp.,* the red yeast *Phaffia rhodozyma,* as well as the bacterium *Paracoccus carotinifaciens sp. nov..* Astaxanthin is also contained in waste from the shellfish processing industry.

Preferably, the biomass comprising astaxanthin of step a. comprises at least one microorganism selected from the group consisting of an alga, a yeast, and a bacterium, at least a waste from the fish supply chain, or a mixture thereof.

Preferably, said alga is selected from the group consisting of *Haematococcus pluvialis, Chlorella vulgaris, Chlorella zoofingiensis* and *Chlorococcum sp..*

Preferably, said yeast is *Phaffia rhodozyma.*

Preferably, said bacterium is *Paracoccus carotinifaciens sp.nov..*

Preferably, said waste from the fish supply chain consists of shellfish processing waste (crawfish, prawns), crabs and molluscs (mussels, clams, snails, abalone, oysters, scallops).

Preferably, the biomass comprising astaxanthin of step a. is in a solid form.

Preferably, said biomass has a water content lower than or equal to 80% by weight. Preferably, the linear carboxylic acid used as the solvent in step a. is selected from the group consisting of acetic acid, propanoic acid, butanoic acid, and pentanoic acid.

Preferably, said step a. of extraction is carried out under stirring at a temperature ranging from 15 °C to 45 °C, more preferably for a time ranging from 15 minutes to 24 hours.

Step a. of the method according to the present invention may be carried out in any apparatus known to the person skilled in the art to carry out a solid-liquid extraction. After step a., the method according to the present invention comprises the step b. of separating in the first dispersion of step a. said solution comprising astaxanthin from said exhausted biomass.

Step b. of the method according to the present disclosure may be carried out in any apparatus known to the person skilled in the art to carry out a solid-liquid separation.

Preferably, said step b. of separating is carried out by means of a solid-liquid separation operation selected from the group consisting of decantation, filtration, centrifugation, flotation.

Preferably, said centrifugation is carried out with an acceleration greater than or equal to 2600 g, more preferably for a time of at least 20 minutes.

Preferably, said flotation is carried out with an inert gas, for example nitrogen.

Preferably, said separation step, especially said centrifugation and said flotation, is carried out at a temperature comprised between 15 °C and 45° C, thus advantageously limiting the deterioration of astaxanthin greatly.

After step b., the method according to the present invention comprises step c. of contacting the solution comprising astaxanthin from step b. with a basic aqueous solution. Through step c. a second dispersion is thus obtained comprising a solid comprising astaxanthin and an exhausted aqueous solution.

Preferably, in said step c. said basic aqueous solution has a pH such as to bring the mixture to a pH equal to or greater than 11.

Preferably, in said step c. said basic aqueous solution is an aqueous solution of at least one base, more preferably a base selected from the group consisting of ammonium hydroxide (NH₄OH), sodium hydroxide (NaOH), ammonium bicarbonate (NH₄HCO₃), potassium hydroxide (KOH), sodium bicarbonate (NaHCO₃), sodium carbonate (Na₂CO₃), and sodium acetate (NaCH₃COO).

Preferably, in said step c. said basic aqueous solution is an aqueous solution of at least one base selected from the group consisting of ammonium hydroxide (NH₄OH), sodium hydroxide (NaOH).

In a preferred embodiment, said basic aqueous solution is a sodium hydroxide aqueous solution, and it is added in a molar ratio ranging from 0.4 to 0.8 moles of NaOH for each mole of solvent.

In a preferred embodiment, when said basic aqueous solution is a sodium hydroxide aqueous solution, the linear carboxylic acid used as solvent in step a. is advantageously selected from the group consisting of acetic acid, propanoic acid, butanoic acid, and pentanoic acid.

In a further preferred embodiment, said basic aqueous solution is an ammonium hydroxide aqueous solution, and it is added in a molar ratio ranging from 0.5 to 5 moles of NH₄OH for each mole of solvent.

Downstream of step c., the method according to the present invention comprises step d. of separating in the second dispersion of step c. said solid comprising astaxanthin from said exhausted aqueous solution. In this way, the method allows to obtain astaxanthin in a solid form.

Step d. of the method according to the present disclosure may be carried out in any apparatus known to the person skilled in the art to carry out a solid-liquid separation, which can be the same or different from that used for step b. of the method according to the present invention.

Preferably, in said step d. said solid is separated from said exhausted aqueous solution by means of a solid-liquid separation operation selected from the group consisting of decantation, filtration, centrifugation, flotation.

Preferably, said centrifugation is carried out with an acceleration greater than or equal to 2600 g, more preferably for a time greater than or equal to 20 minutes.

Preferably, said flotation is carried out with an inert gas, for example nitrogen.

Preferably, said separation step, especially said centrifugation or flotation, is carried out at a temperature comprised between 15° C and 45 °C, thus advantageously limiting the deterioration of astaxanthin greatly.

In addition to being simple and competitive to implement, the method according to the present invention also addresses the need to reduce the environmental impact of the method itself, and in particular it offers the possibility of recovery and regeneration, thus preventing the production of waste deriving from the exhausted extraction solvent.

In a preferred embodiment of the method according to the present invention, in fact, in the aforementioned step c. the basic aqueous solution used is an ammonium hydroxide (NH₄OH) aqueous solution, and the method optionally includes, downstream of step d., the further step of:
e. heating the exhausted aqueous solution separated in step d., so as to remove water and ammonia and reobtain a solvent comprising at least one linear carboxylic acid selected from the group consisting of acetic acid, propanoic acid, butanoic acid, pentanoic acid, and hexanoic acid.

The solvent thus reobtained may in fact be advantageously reused in step a. of the method, thus minimizing waste and the production of exhausted method solvents, to be disposed of.

Thanks to the peculiar characteristics of the method according to the present invention, it is possible to obtain astaxanthin in a solid form, with excellent characteristics of stability and compositional quality, starting from a biomass.

In a further aspect thereof, the present disclosure therefore also relates to the astaxanthin in a solid form obtainable by the method according to the first aspect of the invention.

Said product may in fact be advantageously used for preparing various types of products, such as pharmaceuticals, nutraceuticals, cosmetics, as well as products for human nutrition or animal feed, which benefit from the properties of astaxanthin.

In a still further aspect thereof, the present disclosure therefore also relates to the use of astaxanthin in the form obtainable by the method according to the first aspect of the invention, for the preparation of a pharmaceutical product, a nutraceutical product, a cosmetic product, a product for human nutrition, or a product for animal feed.

The advantages and characteristics of these further aspects have already been highlighted with reference to the first aspect of the invention and are not repeated here.

Further characteristics and advantages of the invention will become more apparent from the following Examples, to be intended for illustrative and non-limiting purposes of the same.

### EXPERIMENTAL PART

### Materials and methods

### Biomass

The biomass consists of dried cells of the bacterium *Paracoccus carotinifaciens* in the form of a dry powder.

### Solvents

CH₃COOH (acetic acid), CH₃CH₂COOH (propanoic acid), CH₃(CH₂)₂COOH (butanoic acid), CH₃(CH₂)₄COOH (esanoic acid), CH₃(CH₂)₆COOH (octanoic acid). The latter was used as a comparison solvent. All solvents, of analytical grade, were supplied by AlfaAesar.

Alkaline solutions of NaOH (sodium hydroxide) were prepared from analytical grade reagents (Carlo Erba, Italy), while NH₄OH (ammonium hydroxide) was available as a 28% aqueous solution (Honeywell Fluka, USA).

### Total extraction via Soxhlet

The content of carotenoids, in particular astaxanthin, in the biomass was evaluated by Soxhlet extraction with acetone. This process was carried out at the acetone boiling point for 24 hours. Subsequently, the solvent, enriched in carotenoids, was subjected to spectrophotometric analysis (Shanghai Mapada Spectrophotometer UV-1800 PC) through the use of a calibration curve of astaxanthin in acetone. Solutions of known concentrations of astaxanthin were prepared and their absorption spectrum was evaluated between 300 and 700 nm. The intensity peak was recorded at 476 nm.

### UV-VIS spectrophotometry

The UV-VIS analysis was carried out with a Shanghai Mapada Spectrophotometer UV-1800 PC) through the use of calibration curves obtained by preparing standard solutions of astaxanthin in the following solvents: CH₃COOH (acetic acid), CH₃CH₂COOH (propanoic acid), CH₃(CH₂)₂COOH (butanoic acid), CH₃(CH₂)₄COOH (esanoic acid), CH₃(CH₂)₆COOH (octanoic acid). Solutions of known concentrations of astaxanthin were prepared and their absorption spectrum was evaluated between 300 and 700 nm. The intensity peak for each solvent was recorded at a wavelength between 477 and 481 nm: 477 nm for CH₃CH₂COOH (propanoic acid) and CH₃(CH₂)₄COOH (esanoic acid), 480 nm for CH₃COOH (acetic acid) and 481 nm for CH₃(CH₂)₂COOH (butanoic acid) and CH₃(CH₂)₆COOH (octanoic acid).

### HPLC

The HPLC analysis on the precipitate was performed on a Waters Alliance e2695 system equipped with a 2489 UV-VIS detector and an Armstrong Inertsil SIL100 column. The elution conditions were as follows: the mobile phase consisted of a water:methanol:dichloromethane:acetonitrile (4.5:28:22:45.5 v/v/v/v) mixture; at a flow rate of 1.0 mL/min; column temperature of 25 °C. Detection and identification were performed using a photodiode array detector (λ_{detection}=476 nm).

### Example 1

0.25 mg of *Paracoccus* biomass was suspended in 7.5 mL of each solvent in a 30 mL Erlenmeyer flask and stirred constantly by magnetic stirring at 25 °C for 4 hours. All extractions were performed in triplicate.

After extraction, the solid-liquid separation was obtained by centrifugation (Mod Thermo Scientific CL10, 20 min at 2600 g) and the supernatant was analyzed by UV-VIS spectrophotometry, according to the method described above.

To recover the extracted astaxanthin, the extract was transferred into a glass container, and a sodium hydroxide aqueous solution (14% w/v) was added in a molar ratio equal to 0.63 moles of NaOH: 1 mole of solvent.

The extract was mixed with the alkali solution and kept under magnetic stirring for 20 min at 25 °C. A dark red solid precipitate formed, and the remaining exhausted aqueous solution turned pale reddish. The precipitate was easily separated from the exhausted aqueous solution by centrifugation (Mod Thermo Scientific CL10, 10 min at 2600 g), thus recovering astaxanthin in solid form.

To quantify the extraction of astaxanthin, after separation of the dark red solid precipitate, the exhausted aqueous solution was analyzed by UV-VIS spectrophotometry, according to the method described above.

The extraction efficiency (EE%) of astaxanthin, obtained by Soxhlet extraction with acetone as previously described, was evaluated from the difference between astaxanthin (AXextracted) in the supernatant after extraction and astaxanthin in the biomass (AXbiomass), according to the following equation: $EE% = \frac{AX extracted}{AXbiomass} \times 100$

The precipitate was then subjected to analysis too. It was resuspended in acetone and analyzed to characterize its astaxanthin content by HPLC according to the above method.

The recovery efficiency (RE%) of astaxanthin was evaluated as the ratio between the amount of astaxanthin in the supernatant after precipitation (AXresidual) divided by the amount initially contained in the supernatant after extraction (AXextracted), according to the following equation: $RE% = \frac{AXresidual}{AX extracted} \times 100$

The results obtained are shown in Table 1:

| Solvent | EE (%) | RE (%) |
|---|---|---|
| CH₃COOH (acetic acid) | 43.10 | 98.87 |
| CH₃CH₂COOH (propanoic acid), | 48.33 | 99.90 |
| CH₃(CH₂)₂COOH (butanoic acid), | 46.81 | 99.63 |
| CH₃(CH₂)₄COOH (esanoic acid), | 45.67 | - |
| CH₃(CH₂)₆COOH (octanoic acid) - comparative example | 30,74 | * |

| | | |
|---|---|---|
| * Astaxanthin could not be recovered from the solution | | |

From the data obtained it was possible to highlight that the fundamental aspect characterizing the best performance of the acids used as extracting agents, object of the present invention, is that they allow astaxanthin to be released after having extracted it, while octanoic acid does not allow astaxanthin to be separated from the solvent, once extracted.

### Example 2

Example 1 was repeated using an aqueous solution of NH₄OH (28% w/v) in a molar ratio equal to 4 moles of NH₄OH:1 mole of acid solvent instead of the sodium hydroxide aqueous solution in a molar ratio of 0.63 moles of NaOH: 1 mole of solvent.

The results relating to the extraction and recovery of astaxanthin from the starting biomass are shown in Table 2.

| Solvent | EE (%) | RE (%) |
|---|---|---|
| CH₃COOH (acetic acid) | 43.10 | 97.46 |
| CH₃CH₂COOH (propanoic acid), | 48.33 | 99.93 |
| CH₃(CH₂)₂COOH (butanoic acid), | 46.81 | 99.95 |
| CH₃(CH₂)₄COOH (esanoic acid), | 45.67 | 99.90 |
| CH₃(CH₂)₆COOH (octanoic acid) - comparative example | 30.74 | * |

| | | |
|---|---|---|
| * Astaxanthin could not be recovered from the solution | | |

As also noted in Example 1, it was apparent that, in addition to the extraction of astaxanthin from the biomass, the solvents of the process according to the present invention allowed also the substantially quantitative recovery of the same from the extract, unlike octanoic acid.

## Claims

1. A method of obtaining astaxanthin from a biomass, comprising the steps of:
a. extracting an astaxanthin-containing biomass with a solvent comprising at least one linear carboxylic acid selected from the group consisting of acetic acid, propanoic acid, butanoic acid, pentanoic acid, and hexanoic acid, thus obtaining a first dispersion comprising an exhausted biomass and a solution comprising astaxanthin;
b. separating in the first dispersion of step a. said solution comprising astaxanthin from said exhausted biomass;
c. contacting the solution comprising astaxanthin from step b. with a basic aqueous solution, thus obtaining a second dispersion comprising a solid comprising astaxanthin and an exhausted aqueous solution; and
d. separating in the second dispersion of step c. said solid comprising astaxanthin from said exhausted aqueous solution, thus obtaining astaxanthin in solid form.

2. The method according to claim 1, wherein said biomass comprises at least one microorganism selected from the group consisting of an alga, a yeast, and a bacterium, at least a waste from the fish supply chain, or a mixture thereof.

3. The method according to claim 2, wherein said alga is selected from the group consisting of *Haematococcus pluvialis, Chlorella vulgaris, Chlorella zoofingiensis* and *Chlorococcum sp..*

4. The method according to claim 2 or 3, wherein said yeast is *Phaffia rhodozyma.*

5. The method according to anyone of claims 2 to 4, wherein said bacterium is *Paracoccus carotinifaciens sp. nov..*

6. The method according to anyone of claims 1 to 5, wherein said biomass has a water content lower than or equal to 80% by weight.

7. The method according to anyone of claims 1 to 6, wherein in said step a. said solvent comprises at least one linear carboxylic acid selected from the group consisting of acetic acid, propanoic acid, butanoic acid, and pentanoic acid.

8. The method according to anyone of claims 1 to 7, wherein said step a. of extracting is carried out under stirring at a temperature ranging from 15 °C to 45 °C.

9. The method according to anyone of claims 1 to 8, wherein in said step c. said basic aqueous solution has a pH such as to bring the mixture to a pH equal to or greater than 11.

10. The method according to anyone of claims 1 to 9, wherein in said step c. said basic aqueous solution is an aqueous solution of at least one base selected from the group consisting of ammonium hydroxide (NH₄OH), sodium hydroxide (NaOH), ammonium bicarbonate (NH₄HCO₃), potassium hydroxide (KOH), sodium bicarbonate (NaHCO₃), sodium carbonate (Na₂CO₃), and sodium acetate (NaCH₃COO).

11. The method according to anyone of claims 1 to 10, wherein said basic aqueous solution is an ammonium hydroxide aqueous solution, and it is added in a molar ratio ranging from 0.5 to 5 moles of NH₄OH for each mole of solvent.

12. The method according to anyone of claims 1 to 10, wherein said basic aqueous solution is a sodium hydroxide aqueous solution, and it is added in a molar ratio ranging from 0.4 to 0.8 moles of NaOH for each mole of solvent.

13. The method according to anyone of claims 1 to 11, wherein in said step c. said basic aqueous solution is an ammonium hydroxide (NH₄OH) aqueous solution, and the method includes the further step of:
e. heating the exhausted aqueous solution separated in step d., so as to remove water and ammonia and reobtain a solvent comprising at least one linear carboxylic acid selected from the group consisting of acetic acid, propanoic acid, butanoic acid, pentanoic acid, and hexanoic acid.

## Patentansprüche

1. Verfahren zur Gewinnung von Astaxanthin aus einer Biomasse, umfassend die Schritte:
a. Extrahieren einer Astaxanthin enthaltenden Biomasse mit einem Lösungsmittel, das mindestens eine lineare Carbonsäure umfasst, die ausgewählt ist aus der Gruppe, bestehend aus Essigsäure, Propansäure, Butansäure, Pentansäure und Hexansäure, wodurch eine erste Dispersion erhalten wird, die eine erschöpfte Biomasse und eine Astaxanthin umfassende Lösung umfasst;
b. Trennen der Astaxanthin umfassenden Lösung von der erschöpften Biomasse in der ersten Dispersion aus Schritt a.;
c. Inkontaktbringen der Astaxanthin umfassenden Lösung aus Schritt b. mit einer basischen wässrigen Lösung, wodurch eine zweite Dispersion erhalten wird, die einen Astaxanthin umfassenden Feststoff und eine erschöpfte wässrige Lösung umfasst; und
d. Trennen des Astaxanthin umfassenden Feststoffs von der erschöpften wässrigen Lösung in der zweiten Dispersion aus Schritt c., wodurch Astaxanthin in fester Form erhalten wird.

2. Verfahren nach Anspruch 1, wobei die Biomasse mindestens einen Mikroorganismus umfasst, der ausgewählt ist aus der Gruppe, bestehend aus einer Alge, einer Hefe und einem Bakterium, mindestens einem Abfall aus der Fischlieferkette oder einer Mischung davon.

3. Verfahren nach Anspruch 2, wobei die Alge ausgewählt ist aus der Gruppe, bestehend aus *Haematococcus pluvialis, Chlorella vulgaris, Chlorella zoofingiensis* und *Chlorococcum sp..*

4. Verfahren nach Anspruch 2 oder 3, wobei die Hefe *Phaffia rhodozyma* ist.

5. Verfahren nach einem der Ansprüche 2 bis 4, wobei das Bakterium *Paracoccus carotinifaciens sp. nov.* ist.

6. Verfahren nach einem der Ansprüche 1 bis 5, wobei die Biomasse einen Wassergehalt von weniger als oder gleich 80 Gew.-% aufweist.

7. Verfahren nach einem der Ansprüche 1 bis 6, wobei das Lösungsmittel in dem Schritt a. mindestens eine lineare Carbonsäure umfasst, die ausgewählt ist aus der Gruppe, bestehend aus Essigsäure, Propansäure, Butansäure und Pentansäure.

8. Verfahren nach einem der Ansprüche 1 bis 7, wobei der Schritt a. des Extrahierens unter Rühren bei einer Temperatur im Bereich von 15 °C bis 45 °C durchgeführt wird.

9. Verfahren nach einem der Ansprüche 1 bis 8, wobei die basische wässrige Lösung in dem Schritt c. einen solchen pH-Wert aufweist, dass die Mischung auf einen pH-Wert von 11 oder mehr gebracht wird.

10. Verfahren nach einem der Ansprüche 1 bis 9, wobei die basische wässrige Lösung in dem Schritt c. eine wässrige Lösung von mindestens einer Base ist, die ausgewählt ist aus der Gruppe, bestehend aus Ammoniumhydroxid (NH₄OH), Natriumhydroxid (NaOH), Ammoniumbicarbonat (NH₄HCO₃), Kaliumhydroxid (KOH), Natriumbicarbonat (NaHCO₃), Natriumcarbonat (Na₂CO₃) und Natriumacetat (NaCH₃COO).

11. Verfahren nach einem der Ansprüche 1 bis 10, wobei die basische wässrige Lösung eine wässrige Ammoniumhydroxidlösung ist und in einem Molverhältnis im Bereich von 0,5 bis 5 Mol NH₄OH pro Mol Lösungsmittel zugegeben wird.

12. Verfahren nach einem der Ansprüche 1 bis 10, wobei die basische wässrige Lösung eine wässrige Natriumhydroxidlösung ist und in einem Molverhältnis im Bereich von 0,4 bis 0,8 Mol NaOH pro Mol Lösungsmittel zugegeben wird.

13. Verfahren nach einem der Ansprüche 1 bis 11, wobei die basische wässrige Lösung in dem Schritt c. eine wässrige Ammoniumhydroxidlösung (NH₄OH) ist und das Verfahren den weiteren Schritt umfasst:
e. Erhitzen der in Schritt d. abgetrennten erschöpften wässrigen Lösung, um Wasser und Ammoniak zu entfernen und ein Lösungsmittel rückzugewinnen, das mindestens eine lineare Carbonsäure umfasst, die ausgewählt ist aus der Gruppe, bestehend aus Essigsäure, Propansäure, Butansäure, Pentansäure und Hexansäure.

## Revendications

1. Procédé d'obtention d'astaxanthine à partir d'une biomasse, comprenant les étapes consistant à :
a. extraire une biomasse contenant de l'astaxanthine avec un solvant comprenant au moins un acide carboxylique linéaire choisi dans le groupe constitué par l'acide acétique, l'acide propanoïque, l'acide butanoïque, l'acide pentanoïque et l'acide hexanoïque, obtenant ainsi une première dispersion comprenant une biomasse épuisée et une solution comprenant de l'astaxanthine ;
b. séparer dans la première dispersion de l'étape a. ladite solution comprenant de l'astaxanthine de ladite biomasse épuisée ;
c. mettre en contact la solution comprenant de l'astaxanthine de l'étape b. avec une solution aqueuse basique, obtenant ainsi une deuxième dispersion comprenant un solide comprenant de l'astaxanthine et une solution aqueuse épuisée ; et
d. séparer dans la deuxième dispersion de l'étape c. ledit solide comprenant de l'astaxanthine de ladite solution aqueuse épuisée, obtenant ainsi de l'astaxanthine sous forme solide.

2. Procédé selon la revendication 1, dans lequel ladite biomasse comprend au moins un micro-organisme choisi dans le groupe constitué par une algue, une levure et une bactérie, au moins un déchet de la chaîne d'approvisionnement en poisson, ou un mélange de ceux-ci.

3. Procédé selon la revendication 2, dans lequel ladite algue est choisie dans le groupe constitué par *Haematococcus pluvialis, Chlorella vulgaris, Chlorella zoofingiensis* et *Chlorococcum sp..*

4. Procédé selon la revendication 2 ou 3, dans lequel ladite levure est *Phaffia rhodozyma.*

5. Procédé selon l'une quelconque des revendications 2 à 4, dans lequel ladite bactérie est *Paracoccus carotinifaciens sp. nov..*

6. Procédé selon l'une quelconque des revendications 1 à 5, dans lequel ladite biomasse a une teneur en eau inférieure ou égale à 80 % en poids.

7. Procédé selon l'une quelconque des revendications 1 à 6, dans lequel dans ladite étape a. ledit solvant comprend au moins un acide carboxylique linéaire choisi dans le groupe constitué par l'acide acétique, l'acide propanoïque, l'acide butanoïque et l'acide pentanoïque.

8. Procédé selon l'une quelconque des revendications 1 à 7, dans lequel ladite étape a. d'extraction est effectuée sous agitation à une température allant de 15 °C à 45 °C.

9. Procédé selon l'une quelconque des revendications 1 à 8, dans lequel dans ladite étape c. ladite solution aqueuse basique a un pH de manière à amener le mélange à un pH égal ou supérieur à 11.

10. Procédé selon l'une quelconque des revendications 1 à 9, dans lequel dans ladite étape c. ladite solution aqueuse basique est une solution aqueuse d'au moins une base choisie dans le groupe constitué par l'hydroxyde d'ammonium (NH₄OH), l'hydroxyde de sodium (NaOH), le bicarbonate d'ammonium (NH₄HCO₃), l'hydroxyde de potassium (KOH), le bicarbonate de sodium (NaHCO₃), le carbonate de sodium (Na₂CO₃), et l'acétate de sodium (NaCH₃COO).

11. Procédé selon l'une quelconque des revendications 1 à 10, dans lequel ladite solution aqueuse basique est une solution aqueuse d'hydroxyde d'ammonium, et elle est ajoutée dans un rapport molaire allant de 0,5 à 5 moles de NH₄OH pour chaque mole de solvant.

12. Procédé selon l'une quelconque des revendications 1 à 10, dans lequel ladite solution aqueuse basique est une solution aqueuse d'hydroxyde de sodium, et elle est ajoutée dans un rapport molaire allant de 0,4 à 0,8 mole de NaOH pour chaque mole de solvant.

13. Procédé selon l'une quelconque des revendications 1 à 11, dans lequel dans ladite étape c. ladite solution aqueuse basique est une solution aqueuse d'hydroxyde d'ammonium (NH₄OH), et le procédé comprend l'étape ultérieure consistant à :
e. chauffer la solution aqueuse épuisée séparée dans l'étape d., de manière à éliminer l'eau et l'ammoniac et à réobtenir un solvant comprenant au moins un acide carboxylique linéaire choisi dans le groupe constitué par l'acide acétique, l'acide propanoïque, l'acide butanoïque, l'acide pentanoïque et l'acide hexanoïque.
